Europäisches Patentamt

European Patent Office

Office européen des brevets

(11) Publication number: **0 406 545 A2**

# EUROPEAN PATENT APPLICATION

(21) Application number: 90109060.5

(22) Date of filing: 14.05.90

(51) Int. Cl.5: **C12N 15/31, A23L 3/34, A01N 63/00**

(30) Priority: 03.07.89 US 375344

(43) Date of publication of application:
09.01.91 Bulletin 91/02

(84) Designated Contracting States:
AT BE CH DE DK ES FR GB GR IT LI LU NL SE

(71) Applicant: MICROLIFE TECHNICS, INC.
1833 57th Street
Sarasota, Florida 34230(US)

(72) Inventor: Vandenbergh, Peter A.
4414 Meadowcreek Circle
Sarasota, Florida 33583(US)
Inventor: Pucci, Michael J.
126 Neanda St.
New Britain, Connecticut 06053(US)
Inventor: Gonzalez, Carlos F.
1203 Pershing Avenue
College Station, Texas 77840(US)

(74) Representative: Patentanwälte Grünecker,
Kinkeldey, Stockmair & Partnerner
Maximilianstrasse 58
D-8000 München 22(DE)

(54) Cloned gene encoding for bacteriocin from pediococcus acidilactici.

(57) Isolation of a gene encoding for a bacteriocin in Pediococcus acidilactici cloning of the gene in a vector plasmid and transformation to Escherichia coli is described. The bacteriocin is particularly useful for inhibiting Listeria and Salmonella in food products.

FIGURE 1

EP 0 406 545 A2

## CLONED GENE ENCODING FOR BACTERIOCIN FROM PEDIOCOCCUS ACIDILACTICI

Cross Reference to Related Application

This application is a continuation-in-part of co-pending application Serial No. 012,619, filed February 9, 1987.

BACKGROUND OF THE INVENTION

(1) SUMMARY OF THE INVENTION

The present invention relates to a method for isolating a gene encoding for a bacteriocin from Pediococcus acidilactici and cloning the gene into a vector which is transformed into Escherichia coli . In particular, the present invention relates to a gene encoding for a bacteriocin derived from a plasmid in Pediococcus acidilactici .

(2) Prior Art

The pediococci are a diverse group of Gram-positive homofermentative lactic acid bacteria often found as saphrophytes on vegetable material (Gonzalez, C. F., and B. S. Kunka, Appl. Environ. Microbiol. 53 :2534-2538 (1987); and Mundt, J. O., W. G. Beattie, and F. R. Wieland, J. Bacteriol. 98 :938-942 (1969)). Commercially, pediococci are used in the fermentation of vegetables (Pederson, Bacteriol. Rev. 13 :225-232 (1949) and meats (Smith, J. L., and S. A. Palumbo, J. Food Prot. 46 :997-1006 (1983).

Some strains of P . pentosaceus and P . acidilactici have been found to contain resident plasmids although the roles of most of these remain unknown (Gonzalez, C. F., and B. S. Kunka, Appl. Environ. Microbiol. 46 :81-89 (1983); Graham, D. C., and L. L. McKay, Appl. Environ. Microbiol. 50 :532-534 (1985); and Raccach, M., CRC Crit. Rev. Microbiol. 14 :291-309 (1987). Recently, the association of raffinose fermentation and plasmid DNA has been reported (Gonzalez, C. F., and B. S. Kunka, Appl. Environ. Microbiol. 51 :105-109 (1986). There have also been several reports which associate the production of bacteriocins with host plasmid DNA (Daeschel, M. A., and T. R. Klaenhammer, Appl. Environ. Microbiol. 51 :1538-1541 (1985); Gonzalez, C. F., and B. S. Kunka, Appl. Environ. Microbiol. 53 :2534-2538 (1987); Graham, D. C., and L. McKay, Appl. Environ. Microbiol. 50 :532-534 (1985); and Bhunia et al, J. Applied Bact. 65 :261-268 (1988)). The cloning of genes for the production of the bacteriocin has not been described and this would be useful for producing bacteriocin in significant quantities in genera unrelated to Pediococcus .

Cloned Gram-positive genes for different unrelated proteins have been shown to express in Escherichia coli (Gilmore, M. S., Curr. Top. Microbiol. Immunol. 118 :219-234 (1985); Rogeson, J. P., R. G. Barletta, and R. Curtiss III, J. Bacteriol. 153 :211-221 (1983); and Smorawinska, M., J. C. Hsu, J. B. Hansen, E. K. Jagusztyn-Krynicka, YH. Abiko, and R. Curtiss III, J. Bacteriol. 153 :1095-1097 (1983)).

OBJECTS

It is therefore an object of the present invention to provide an isolated and purified gene segment which is cloned into a vector plasmid and expressed in a bacterium, preferably unrelated to the genus Pediococcus , so that the bacteriocin can be produced in a broad spectrum of genera of bacteria. Further, it is an object to produce the bacteriocin for use in foods and the like to inhibit spoilage bacteria, Listeria , Salmonella and Pediococcus . These and other objects will become increasingly apparent by reference to the following description and the drawings.

IN THE DRAWINGS

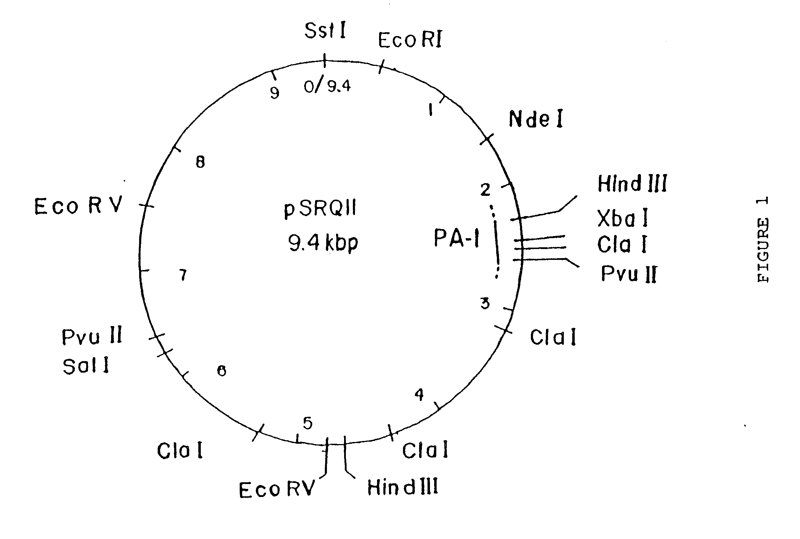

Figure 1 shows a restriction endonuclease site map of pSRQ11. P. acidilactici PAC1.0 plasmid pSRQ11

2

is 9.4 kbp and contains the gene for PA-1 bacteriocin. The approximate position of the PA-1 gene containing restriction endonuclease sites Hin dIII, Xba I, Cla I, and Pvu II is shown.

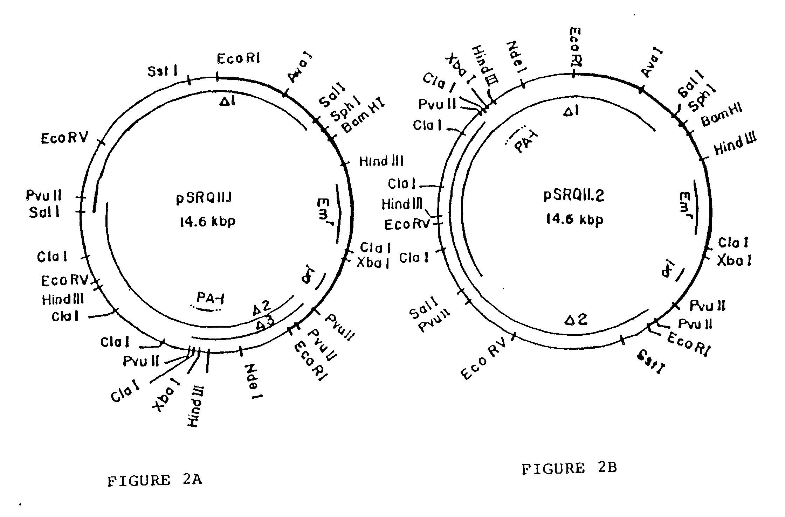

Figures 2A and 2B show restriction endonuclease site maps of pSRQ11.1 and pSRQ11.2. Both plasmids are 14.6 kbp and contain erythromycin resistance (Em') genes at the locations indicated. The Escherichia coli origin of replication (ori) and the PA-1 bacteriocin gene position on each plasmid are shown. Numbered triangles ( 1Δand Δ2) indicate areas of each plasmid which had been subsequently deleted. Heavy shaded lines indicate Escherichia coli plasmid pVA891 portions while the remainder of the plasmids are PAC1.0 pSRQ11 DNA.

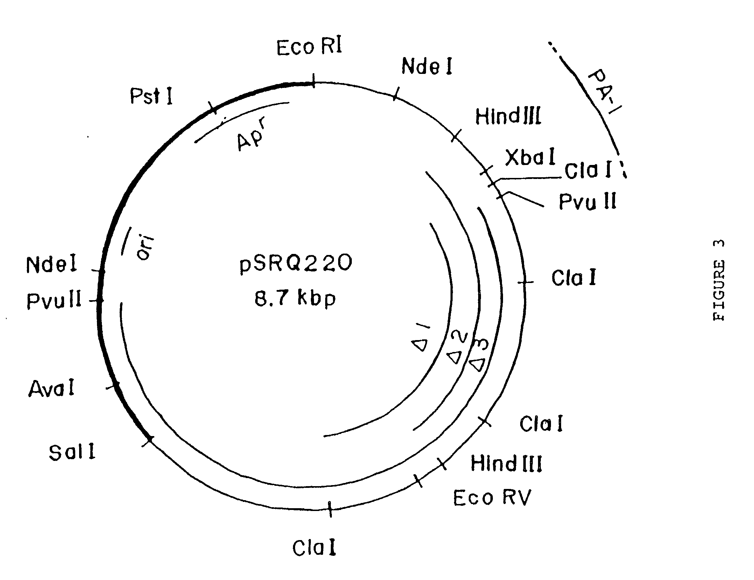

Figure 3 shows a restriction endonuclease site map of PSRQ220. Plasmid PSRQ220 is 8.7 kbp and is a chimera of Escherichia coli plasmid pBR322 and PAC1.0 plasmid pSRQ11 digested with Eco RI and Sal I and ligated together. The heavy shaded line indicates the pBR322 portion of plasmid. The Escherichia coli origin of replication (ori), ampicillin resistance (Ap') gene, and the approximate location of the PA-1 bacteriocin gene are indicated. Regions of the plasmid which were subsequently deleted are denoted by numbered triangles (Δ1, Δ2 and Δ3)

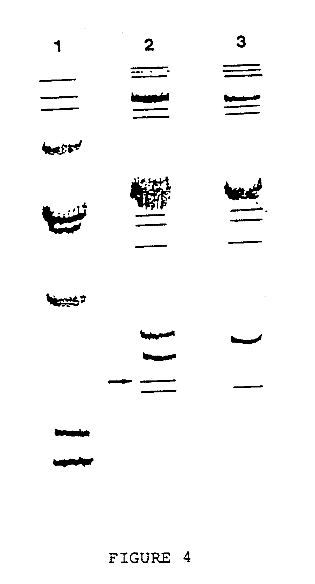

Figure 4 shows SDS-PAGE profiles of PAC1.0 and PAC1.14 extracellular proteins. Lane 1 indicates seven molecular mass standards. These are, from top to bottom, 200 kDa, 97 kDa, 68 kDa, 43 kDa, 29 kDa, and 18.4 kDa, and 14.3 kDa. Lane 2 indicates PAC1.0 extracellular proteins while lane 3 displays PAC1.14 extracellular proteins. Arrow indicates the PA-1 polypeptide.

## GENERAL DESCRIPTION

The present invention relates to a gene segment of isolated and purified DNA from a Pediococcus encoding for a polypeptide which is a bacteriocin having a molecular mass of between about 19,000 to 20,000 daltons by SDS-PAGE analysis.

The present invention further relates to a chimeric plasmid for transformation to Escherichia coli , which is Gram-negative and unrelated to Pediococcus , including a gene segment of DNA from Pediococcus encoding for a polypeptide which is a bacteriocin having a molecular mass of between about 19,000 to 20,000 daltons by SDS-PAGE analysis and a plasmid vector for the Escherichia coli linked to the segment so that the bacteriocin is expressed in the Escherichia coli transformed with the chimeric plasmid.

Finally the present invention relates to a transformed Escherichia coli containing a chimeric plasmid including a gene segment of DNA Pediococcus encoding for a polypeptide which is a bacteriocin and having a molecular mass of between about 19,000 and 20,000 daltons by SDS-PAGE analysis and a plasmid vector for the Escherichia coli linked to the segment so that the bacteriocin is expressed by the transformed Escherichia coli .

The gene segment is preferably derived from Pediococcus acidilactici NRRL-B-18050 also known herein as PAC1.0, which is deposited with the Northern Regional Research Laboratory in Peoria, Illinois under the Budapest Treaty. The gene is carried in a 9.4 kbp plasmid designated herein as pSRQ11. A gene segment pSRQ220 ( Sal I to Eco RI; 6kbp) is ligated in purified form in a vector plasmid V871 (pSRQ220) in Escherichia coli NRRL-B-18429 and deposited at the same depository under the Budapest Treaty. Other gene segments of Pediococcus encoding for a bacteriocin can be isolated and cloned into other genera or species of bacteria using procedures well known to those skilled in the art.

U.S. Serial No. 12,619, filed February 9, 1987 and assigned to a common assignee describes the isolation of a bacteriocin from Pediococcus acidilactici NRRL-B-18050. A plasmid in this strain was disclosed to encode for the bacteriocin which was described to be useful in foods to inhibit bacterial spoilage.

U.S. application Serial No. 148,044 assigned to a common assignee describes a method of using the bacteriocin to inhibit Listeria monocytogenes which produces a severe illness in humans. The plasmid pSRQ11 was described as the source of the bacteriocin.

## SPECIFIC DESCRIPTION

The following Examples show the cloning of the gene for bacteriocin PA-1 into a vector, transformation into Escherichia coli and characterization of the bacteriocin produced. The bacteriocin PA-1 produced by a cloned gene from pSRQ11 was found to express in Escherichia coli in both liquid and solid media. By analysis of deletion derivatives, the coding region of the gene for PA-1 was localized to an area encompassing closely clustered Hin dIII, Xba I, Cla I, and Pvu II restriction sites. This was further verified by insertional inactivation via insertion of a DNA fragment into the Xba I site. Removal of the Xba I insert led to

restoration of bacteriocin activity. The approximate molecular mass of a PA-1 bacteriocin polypeptide produced by the gene was found to be 19,000 to 20,000 daltons by comparison with the protein profile from a plasmid-cured derivative on SDS-PAGE slab gels. Partial purification of the bacteriocin was obtained using cation exchange HPLC.

Bacterial strains and media . The bacterial strains used are listed in Table 1.

### Table 1. Bacterial Strains and Plasmids

| Strain or plasmid | Remarks[a] | Reference |
|---|---|---|
| **P. acidilactici** | | |
| PAC1.0 | Contains 9.4kbp PA-1 bacterio-cin plasmid, pSRQ11 | (4) |
| PAC1.14 | PAC1.0 derivative cured of pSRQ11 | (4) |
| **P. pentosaceus** | | |
| FBB63C | Sensitive indicator strain for PA-1 bacteriocin | (4) |
| **E. coli** | | |
| V850 | Hypersensitivity to macrolide antibiotics | (5) |
| V871 | Tetracycline sensitive | (7) |
| **Plasmids** | | |
| pBR322 | $Ap^r$, $Tc^r$ | (1) |
| pACYC184 | $Cm^r$, $Tc^r$ | (2) |
| pVA891 | $Em^r$ | (6) |

| pSA3 | Em$^r$, Cm$^r$, Tc$^r$ | (3) |
|---|---|---|
| pSRQll | 9.4 kbp PA-1 bacteriocin plasmid | (4) |
| pSRQ11.1 | pSRQll in EcoRI site of pVA891; bac$^+$ | present invention |
| pSRQ11.2 | pSRQll in EcoRI; opposite orientation from pSRQll.1; bac$^+$ | present invention |
| pSRQll.11 | SalI deletion derivative of pSRQll.1; bac$^+$ | present invention |
| pSRQll.12 | PvuII deletion derivative of pSRQll.1; bac$^-$ | present invention |
| pSRQll.13 | PvuII deletion derivative of pSRQll.11; bac$^-$ | present invention |
| pSRQll.21 | SalI deletion derivative of pSRQll.2; bac$^-$ | present invention |
| pSRQll.22 | PvuII deletion derivative of pSRQll.2; bac$^-$ | present invention |
| pSRQ161 | pSRQll in EcoRI site of pSA3; bac$^+$ | present invention |
| pSRQ210 | pSRQll XbaI-SalI fragment in XbaI, SalI sites of pACYC184; bac$^-$ | present invention |
| pSRQ211 | pSRQll HindIII fragment c in HindIII site of pACYC184; bac$^-$ | present invention |
| pSRQ220 | pSRQll EcoRI, SalI fragment in EcoRI, SalI sites of pBR322; bac$^+$ | present invention |
| pSRQ220.1 | ClaI deletion derivative of pSRQ220; bac$^-$ | present invention |
| pSRQ220.2 | HindIII deletion derivative of pSRQ220; bac$^-$ | present invention |
| pSRQ220.3 | PvuII deletion derivative of pSRQ220; bac$^-$ | present invention |
| pSRQ221 | pACYC184 in XbaI site of pSRQ220; bac$^-$ | present invention |
| pSRQ221.2 | XbaI deletion of pSRQ221; bac$^+$ | present invention |
| pSRQ222 | pACYC184 XbaI-EcoRI fragment in XbaI, EcoRI sites of pSRQ220; bac$^-$ | present invention |

$^a$Ap$^r$, growth on medium containing >25 ug/ml ampicillin;
Tc$^r$, growth on medium containing >10 ug/ml tetracycline;
Cm$^r$, growth on medium containing >25 ug/ml chloramphenicol;
Em$^r$, growth on medium containing >50 ug/ml erythromycin;
bac$^+$, PA-1 bacteriocin activity; bac$^-$, no PA-1 activity
detected.

(1) Bolivar, F., et al., Gene 2:95-113 (1977).

(2) Chang, A.C.Y., et al., J. Bacteriol.
134:1141-1156 (1978).

(3) Dao, My Lien, et al., Applied and Environmental Microbiology, 49:115-119 (Jan. 1985).

(4) Gonzalez, Carlos F., et al., Applied and Environmental Microbiology, 53:2534-2538 (Oct. 1987).

(5) Macrina, Francis L., et al., Gene, 19:345-353 (1982).

(6) Macrina, Francis L., et al., Gene, 25:145-150 (1983).

(7) Tobian, Janet Ash, et al., Journal of Bacteriology, 160:556-563 (Nov. 1984).

Pediococcus spp. were routinely maintained on MRS agar (Difco Laboratories, Detroit, MI). Escherichia coli strains were routinely carried on Lennox L agar (Gibco/BRL, Gaithersburg, Md.). Escherichia coli strains were also grown on modified MRS agar (no citrate or acetate) or in M9 medium (Maniatis, T., E. F. Fritsch, and J. Sambrook, Molecular cloning: a laboratory manual. Cold Spring Harbor Laboratory, Cold Spring Harbor, NY (1982)) supplemented with 1% yeast extract (Oxoid, Ltd., Basingstoke, Hampshire, U.K.) and 1% Hy CaseTM - (Sheffield Products, Norwich, NY) for bacteriocin assays. Purification studies used FMC (Terleckyj, B., N. P. Willett, and G. D. Shockman, Infect. Immun. 48 :303-311 (1975)) medium supplemented with 2% yeast extract. Selective antibiotic concentrations were as follows: ampicillin, 25 ug/ml; tetracycline, 10 ug/ml; erythromycin, 50 ug/ml; and chloramphenicol, 25 ug/ml. All antibiotics were purchased from Sigma Chemical Co., St. Louis, MO.

Bacteriocin assays . Production of bacteriocin was assayed as previously described (Gonzalez, C. F., and B. S. Kunka, Appl. Environ. Microbiol. 53 :2534-2538 (1987)). Strains were patched on MRS agar or modified MRS agar for Escherichia coli and incubated at 35° C for 18 hours. The plates were then overlaid with soft agar (0.8%) seeded with indicator cells. Isolates which produced a clear, defined zone of inhibition were considered as bacteriocin producers.

Isolation and analysis of plasmid DNA . Covalently closed circular plasmid DNA was isolated from Escherichia coli by the method of Clewell and Helinski (Clewell, D. B., and D. R. Helinski, Biochemistry 59 :4428-4440 (1970)). Escherichia coli strains were screened for plasmid content as previously described (Macrina, F. L., J. A. Tobian, K. R. Jones, R. P. Evans, and D. B. Clewell, Gene 19 :345-353 (1982)). Pediococcus plasmid DNA was obtained by a scaled up modification of the LeBlanc and Lee procedure (LeBlanc, D. J., and L. N. Lee, J. Bacteriol. 140 :1112-1115 (1979)) as described by Gonzalez and Kunka (Gonzalez,C. F., and B. S. Kunka, Appl. Environ. Microbiol. 46 :81-89 (1983)). Plasmid DNA and restriction endonuclease digests were analyzed by agarose gel electrophoresis on 0.8% agarose (Bethesda Research Laboratories, Inc., Gaithersburg, MD) slab gels. Size standards were Escherichia coli V517 (Macrina, F. L., D. J. Kopecko, K. R. Jones, D. J. Ayers, and S. McCowen, Plasmid 1 :417-420 (1978)) for undigested plasmid DNA and Hin dIII -digested bacteriophage lambda DNA (Bethesda Research Laboratories) for restriction endonuclease - cleaved plasmid DNA.

DNA enzymology . Restriction endonuclease digestions were performed in low-, medium-, or high-salt buffers, as recommended by Maniatis et al. (Maniatis, T., E. F. Fritsch, and J. Sambrook, Molecular cloning: a laboratory manual. Cold Spring Harbor Laboratory, Cold Spring Harbor, NY (1982)) . Restriction enzymes were obtained from Bethesda Research Laboratories. DNA ligation reactions were carried out with T4 DNA ligase (Bethesda Research Laboratories) at 4° C for 18 hours according to conditions recommended by the manufacturer.

Bacterial transformations . Escherichia coli was transferred by the CaCl₂ heat shock method Molecular cloning: a laboratory manual. Cold Spring Harbor Laboratory, Cob Spring Harbor, NY (1982)) with cells harvested at an optical density at 660 nm of 0.2 to 0.3.

Sodium dodecyl sulfate - polyacrylamide gel electrophoresis . Polypeptides were separated by sodium dodecyl sulfate (SDS)- polyacrylamide gel electrophoresis (Laemmli, U. K., and M. Favre., J. Mol. Biol. 80 :575-599 (1973)) on a 12% polyacrylamide slab gel with a 4% stacking gel and visualized by Coomassie blue staining. Proteins were boiled for 5 minutes in sodium dodecyl sulfate sample buffer before electrophoresis. Molecular mass standards were purchased from Bethesda Research Laboratories.

Example 1

Restriction endonuclease map of pSRQ11 . The gene encoding the bacteriocin PA-1 was previously shown to be associated with the presence of a 9.4 kilobase plasmid, designated pSRQ11 (Gonzalez, C. F., and B. S. Kunka, Appl. Environ. Microbiol. 53 :2534-2538 (1987)). Plasmid pSRQ11 was digested with a number of restriction endonucleases to generate the restriction site map shown in Figure 1. The plasmid contained several unique sites including Eco RI, Nde I, Xba I, Sal I, and Sst I. Other restriction enzymes which cleaved the plasmid were Cla I, Hin dIII, Pvu II, and Eco RV. The following restriction sites were not found on pSRQ11: Ava I, Bam HI, Sph I, Nru I, Pst I, and Bgl II.

Example 2

Expression of PA-1 bacteriocin in E. coli. Plasmid pSRQ11 was digested with Eco RI and cloned into the Eco RI site on plasmid pVA891 (Macrina, F. L., et al., Gene 25 :145-150 (1983)), which contains an erythromycin resistance marker expressed in both Escherichia coli and streptococci. Recombinant plasmids were obtained with pSRQ11 inserted in both orientations and were designated pSRQ11.1 and pSRQ11.2 as shown in Figure 2. These Escherichia coli strains were assayed for expression of the PA-1 bacteriocin as previously described (Gonzalez, C. F., and B. S. Kunka, Appl. Environ. Microbiol. 53 :2534-2538 (1987)). The strains were grown on modified MRS medium and overlaid with Pediococcus pentosaceus FBB63 indicator strain. Escherichia coli strains containing pSRQ11.1 and pSRQ11.2 both produced zones of inhibition in the indicator lawn while the control Escherichia coli V850 strains showed no zone of inhibition. PA-1 activity by Escherichia coli strains was also observed in liquid medium. Escherichia coli strain V850 carrying pSRQ161 (Table 1) was grown in M9 medium supplemented with 1% yeast extract and 1% Hy Case. After overnight growth, the culture supernatant yielded 400 AU/ml PA-1 bacteriocin activity. This strain grown in the above medium gave the best results of those assayed. Example 2 shows that the cloned PA-1 bacteriocin from Pediococcus acidilactici PAC 1.0 can be expressed and is functional in an Escherichia coli host strain.

Example 3

Deletion derivative analysis of pSRQ11 subclones. In order to localize the region encoding the PA-1 gene, Sal I and Pvu II deletion derivatives of pSRQ11.1 and pSRQ11.2 were obtained (Figure 1). The Sal I deletion of pSRQ11.1 retained activity while the Pvu II deletion derivatives displayed no zones of inhibition against the indicator strain (Table 1). Both the Pvu II and Sal I deletion derivatives of pSRQ11.1 expressed no PA-1 activity (Table 1). These data suggested that the bacteriocin gene was located on the approximately 6.0 kbp Eco RI-Sal I fragment of pSRQ11.1 as shown in Figure 2A.

The 6.0 kbp Eco RI-Sal I fragment then was subcloned into the Eco RI and Sal I restriction sites on the Escherichia coli plasmid, pBR322 (Bolivar, F., R. L. Rodriguez, P. J. Greene, M.C. Betlach, H. L. Heyneker, H.W. Boyer, J. H. Crosa, and S. Falkow, Gene 2 :95-113 (1977)), and the resulting chimeric plasmid was designated pSRQ220 (Figure 3). The Escherichia coli strain containing pSRQ220 was assayed and found to express bacteriocin activity while Cla I, Hin dIII, and Pvu II deletion derivatives (Figure 3) were assayed and found to be negative for PA-1 bacteriocin activity (data not shown). Two additional subclones were obtained: pSRQ210, which consisted of the pSRQ11 Xba I-Sal I fragment cloned into Escherichia coli vector pACYC184 (Chang, A.C.Y., and S. N. Cohen., J. Bacteriol. 134 :1141-1156 (1978)), and pSRQ211, which consisted of pSRQ11 Hin dIII fragment c (from map coordinates 2.3 to 4.6, Figure 1) also cloned into pACYC184. Neither of these two strains expressed PA-1 bacteriocin activity. Together, all of these data suggest that the gene encoding the PA-1 bacteriocin lies in the region of the plasmid containing the closely clustered Hin dIII, Xba I, Cla I, and Pvu II restriction sites (see Figure I) spanning approximately 500 bp of pSRQ11 DNA.

Example 4

Insertional inactivation of the PA-1 bacteriocin gene . Since the Xba I restriction site is unique on both pSRQ11 and pSRQ220 and lies within the PA-1 coding region, it was chosen as a site to insert a foreign DNA fragment and interrupt transcription of the bacteriocin gene. Plasmid pACYC184, approximately 4 kbp in size and also containing a single Xba I site, was cloned into the Xba I site on pSRQ220. The strain containing the resulting recombinant plasmid, pSRQ221, was assayed for PA-1 activity and proved negative (data not shown). When the pACYC184 insert was removed by Xba I digestion followed by religation, activity was once again restored (data not shown). Another construct where the Xba I-Eco RI fragment of pSRQ220 was replaced by the Xba I-Eco RI fragment of pACYC184 also was negative for bacteriocin activity (data not shown). These data indicate that the Xba I site lies within or very close to the coding region of the PA-1 bacteriocin gene.

Example 5

Molecular weight determination of PA-1 bacteriocin . The molecular mass of PA-1 bacteriocin was estimated by SDS-PAGE. Pediococcus acidilactici PAC1.0 and the cured derivative PAC1.14 were grown in the semi-defined FMC-yeast extract medium and Escherichia coli UW-11 with pSRQ161 grown in M9 minimal medium-supplement with 1% by weight lactose, yeast extract and HycaseTM (casein hydrolysate). The two culture supernatants were concentrated by ammonium sulfate precipitation. The buffer resuspended precipitates were dialyzed and run side-by-side on a 12% polyacrylamide slab gel. As shown in Figure 4, a polypeptide of approximately 19,000 to 20,000 daltons molecular mass is present in the UW-11 and PAC1.0 lanes but absent in the lane containing extracellular proteins from the cured derivative, PAC1.14.

Example 6

Cloning of the Eco R1 - Hin dIII fragment 4.0 kbp fragment from pSRQ220 into the expression vector pKK223-3.

The E . coli expression vector (pKK223-3) contains the tac promoter which is repressed but may be derepressed by the addition of isopropyl B-D-thiogalactoside (IPTG). This cloning vector also contains a multiple cloning site which facilitates the insertion of genes behind the promoter and ribosomal binding site. This cloning vector, pKK223-3 (20 ug) was cleaved to completion with the restriction enzymes Eco R1 and then Hin dIII.

The linear Eco R1-Hin dIII fragment was ligated with the plasmid pSRQ220. Prior to ligation the pSRQ220 (75 ug) was first cut to completion with Eco R1 and then partially digested with Hin dIII. The partial Hin dIII digestion of pSRQ220 was achieved by increasing the NaCl concentration in the Hin dIII buffer to 300 mM.

The DNA mixture was allowed to ligate for 18 hours at 16°C then transformed into E . coli JM105. The transformants were selected on media containing carbenicillin (50 ug/ml) and then checked for their ability to produce PA-1. Various transformants were lysed and DNA was purified on a CsCl-Ethidium bromide gradient. The 4.0 kbp Eco R1-Hin dIII-Hin dIII fragment from pSRQ220 was cloned into the Eco R1-Hin dIII site of pKK223-3. This isolate was designated as JM105 (pSRQ225) which produced PA-1. The initial Eco R1-Hin dIII (1.75 kbp) fragment from pSRQ220 was also cloned into pKK223-3 and transformed into E . coli JM105. This isolate designated as JM105 (pSRQ226) did not produce the bacteriocin PA-1.

The molecular mass of the PA-1 protein was previously reported to be approximately 16,500 daltons (Gonzalez, C. F., and B. S. Kunka, Appl. Environ. Microbiol. 53 :2534-2538 (1987)). The estimate was obtained by ascending gel filtration chromatography, which is less accurate. The semi-defined supplemented FMC medium used herein had fewer contaminating peptides than the previously used MRS medium and polypeptide profiles were examined on SDS-PAGE which is more accurate.

As can be seen from the foregoing Examples, Pediococcus acidilactici PAC1.0 produces a bacteriocin designated PA-1, which is plasmid-encoded. The results presented in the Examples map the position of the PA-1 bacteriocin gene on the 9.4 kilobase plasmid, pSRQ11. Deletion derivative analysis of the Escherichia coli - Pediococcus chimerics localized the gene to an area of clustered restriction endonuclease sites within an approximately 500 bp span between Hind III and Pvu II (about 2.3 kbp to 2.8 kbp on Figure 1). Included in this cluster of restriction sites was a unique Xba I site.

As a further verification of the proposed location of the PA-1 gene, plasmid pACYC184 was digested with Xba I and inserted into the unique Xba I site of pSRQ220. The resulting chimeric plasmid, pSRQ221, when transformed back into Escherichia coli , was found to be bacteriocin negative. When the Xba I fragment was removed by Xba I digestion followed by religation, the strain containing this recombinant plasmid was once again producing PA-1 bacteriocin. These data indicate that the Xba I site lies within or very near the PA-1 coding region.

Escherichia coli strains containing chimeric plasmids pSRQ161, pSRQ220, or others (see Table 1) were shown to be capable of PA-1 expression on plate assays. Activity was experienced in Escherichia coli culture supernatant although reduced from that seen in PAC1.0 culture supernatants. The examples show that PA-1 bacteriocin is produced and expressed when cloned into Escherichia coli .

The bacteriocin PA-1 was found to inhibit food sspoilage bacteria, Listeria monocytogenes and Salmonella newport . It is as useful in foods as is the bacteriocin from the uncloned gene.


## Claims

1. A gene segment of isolated and purified DNA from a Pediococcus encoding for a polypeptide which is a bacteriocin having a molecular mass of between about 19,000 to 20,000 daltons by SDS-PAGE analysis.

2. The gene segment of Claim 1 wherein the Pediococcus is Pediococcus acidilactici .

3. The gene segment of Claim 1 derived from Pediococcus acidilactici NRRL-B-18050 as the Pediococcus.

4. The gene segment of Claim 1 derived from plasmid pSRQ11 as carried in Pediococcus acidilactici NRRL-B-18050.

5. The gene segment of Claim 4 wherein the pSRQ11 is digested with Eco R$_1$ as a restriction enzyme to provide the gene segment.

6. The gene segment of Claim 1 as carried in Escherichia coli NRRL-B-18429.

7. A chimeric plasmid for transformation to Escherichia coli including a gene segment of DNA from Pediococcus encoding for a polypeptide which is a bacteriocin having a molecular mass of between about 19,000 to 20,000 daltons by SDS-PAGE analysis and a plasmid vector for the Escherichia coli linked to the segment so that the bacteriocin is expressed in the Escherichia coli transformed with the vector plasmid.

8. The chimeric plasmid of Claim 7 wherein the Pediococcus is Pediococcus acidilactici .

9. The chimeric plasmid of Claim 7 wherein the gene is derived from Pediococcus acidilactici NRRL-B-18050 as the Pediococcus .

10. The chimeric plasmid of Claim 7 wherein the gene segment is derived from plasmid pSRQ11 as carried in Pediococcus acidilactici NRRL-B-18050.

11. The chimeric plasmid of Claim 10 wherein the plasmid pSRQ11 is digested with Eco R$_1$ as a restriction enzyme to provide the gene segment.

12. The chimeric plasmid of Claim 7 as carried in Escherichia coli NRRL-B-18429.

13. A transformed Escherichia coli containing a chimeric plasmid including a gene segment of DNA Pediococcus encoding for a polypeptide which is a bacteriocin having a molecular mass of between about 19,000 to 20,000 daltons by SDS-PAGE analysis and a plasmid vector for the Escherichia coli linked to the segment so that the bacteriocin is expressed by the transformed Escherichia coli .

14. The transformed Escherichia coli of Claim 13 wherein the gene is derived from Pediococcus acidilactici NRRL-B-18050 as the Pediococcus .

15. The transformed Escherichia coli of Claim 13 wherein the gene segment is derived from plasmid pSRQ11 as carried in Pediococcus acidilactici NRRL-B-18050.

16. The transformed Escherichia coli of Claim 15 wherein the plasmid pSRQ11 is digested with Eco R$_1$ as a restriction enzyme to provide the gene segment.

17. Transformed Escherichia coli NRRL-B-18429.

18. The purified bacteriocin produced by the gene of Claim 1 which inhibits Listeria monocytogenes , Salmonella newport , and Lactobacillus bifermentans .

19. The purified bacteriocin produced by the bacterium of Claim 13 which inhibits Listeria monocytogenes , Salmonella newport and Lactobacillus bifermentans .

20. A gene segment of isolated and purified DNA from a Pediococcus encoding for a polypeptide produced from a plasmid designated as pSRQ11 and carried in Pediococcus acidilactici NRRL-B-18050.

21. A chimeric plasmid for transformation to E . coli including a gene segment of isolated and purified DNA from a Pediococcus encoding for a polypeptide produced from a plasmid designated as pSRQ11 and carried in Pediococcus acidilactici NRRL-B-18050 and a plasmid vector for the E . coli linked to the segment so that the bacteriocin is expressed in the E . coli transformed with the chimeric plasmid.

22. A transformed E . coli containing a chimeric plasmid including a gene segment of isolated and purified DNA from a Pediococcus encoding for a polypeptide produced from a plasmid designated as pSRQ11 and carried in Pediococcus acidilactici NRRL-B-18050 and a plasmid vector for the E . coli linked to the segment so that the bacteriocin is expressed in the Escherichia coli transformed with the chimeric plasmid.

FIGURE 1

FIGURE 2B

FIGURE 2A

12

FIGURE 3

FIGURE 4